# EUROPEAN PATENT SPECIFICATION

(11) **EP 1 454 600 B1**
(45) Date of publication and mention of the grant of the patent: **31.08.2011**
(21) Application number: 02780078.8
(22) Date of filing: 13.11.2002
(51) Int. Cl.: A61F 2/14, A61L 27/38, A61L 27/54

(54) **ECTOCORNEA-LIKE SHEET AND METHOD OF CONSTRUCTING THE SAME**
EKTOKORNEA-ARTIGES BLATT UND HERSTELLUNGSVERFAHREN DAFÜR
FEUILLE DU TYPE EPITHELIUM CORNEEN ET PROCEDE DE CONSTRUCTION ASSOCIE

(30) Priority: 19.11.2001 JP 2001352675
(43) Date of publication of application: 08.09.2004
(73) Proprietor: ArBlast Co., Ltd., Kobe Hyogo 650-0047 (JP)
(72) Inventor: NAKAMURA, Takahiro, Kyoto-shi, Kyoto 616-8304 (JP); KINOSHITA, Shigeru, Osaka-shi, Osaka 545-0035 (JP)
(74) Representative: Müller Fottner Steinecke
(86) International application number: PCT/JP2002/011857
(87) International publication number: WO 2003/043542

(56) References cited:
- WO-A1-01/80760
- US-A- 5 015 584
- US-A- 5 032 508
- US-A- 5 885 829
- US-A- 6 143 315
- UEDA M: "Formation of epithelial sheets by serially cultivated human mucosal cells and their applications as a graft material." NAGOYA JOURNAL OF MEDICAL SCIENCE, vol. 58, 1 March 1995 (1995-03-01), pages 13-28, XP002570389 Japan ISSN: 0027-7622

## Description

### TECHNICAL FIELD

The present invention relates to a corneal epithelium (ectocornea)-like sheet, a corneal epithelium (ectocornea) transplantation sheet, and a method of constructing the same. The present invention can be used for treating diseases (ocular surface diseases) that need transplantation of corneal epithelium. Particularly, the present invention provides an effective means for treating corneal disease occurring in bilateral eyes.

### BACKGROUND ART

In surgical treatment for ocular surface disease in which the cornea is covered with the conjunctival epithelium to cause haze, at the present time, corneal epithelium transplantation is used. However, in refractory keratoconjunctivitis with severe inflammation (Setevens-Johonson syndrome, ocular cicatricial pemphigoid, corneal corrosion, and the like), the prognosis appears extremely poor. The biggest reason therefor is thought to be because the corneal epithelium from a different system (allo) having a strong antigenicity is recognized and immunologically rejected by the immune system of a host. In addition, complication caused by systemic and local administration of a large amount of immunosuppressive drug for preventing the rejection after the operation is one of the largest factors for bad prognosis. On the other hand, when the corneal epithelium of allo is used, there is a problem as to the shortage of donor. Therefore, it is thought that transplantation using autologous corneal epithelium tissue is ideal. In the case of unilateral eye disease (such as corneal corrosion), there is a report that the corneal epithelium from the normal eye could be successfully transplanted to the affected eye. However, most of the intractable corneal diseases are bilateral-eye diseases, so that the above-mentioned technique actually cannot be used.

### DISCLOSURE OF INVENTION

With the foregoing in mind, it is an object of the present invention to provide a corneal epithelium-like sheet, a corneal epithelium transplantation sheet, and a method of constructing the same. In particular, it is an object to provide a transplantation material capable of being used for treating patients who have difficulty in obtaining autologous corneal stem cells.

The present inventors have investigated in view of the WO/0180760 A1 discloses: a corneal epithelium-like sheet, comprising a cell layer comprising a layered structure of epithelial cells of limbal (corneal) origin.

Ueda M.; Nagoya. Med. Sci., 58,13-28, 1995 discloses: skin grafts obtained by epithelial cells of mucosal origin comprising a cell layer comprising a layered structure. above-mentioned problems. Firstly, since in the patients with bilateral-eye disease, autologous corneal stem cells cannot be obtained, the present inventors have thought it ideal to use an autologous mucosal tissue of the other normal sites and selected an oral mucosal epithelium as a cell source. This is because it is suggested that the oral mucosal epithelium has stem cells in the local mucosal tissue and it has low differentiation and high divisional potential, and is more easily available as compared with the epidermis that is other epithelial tissue. On the other hand, the present inventors have employed amniotic membrane as a substrate for culturing the oral mucosal epithelial cell.

Firstly, an oral mucosal epithelium was collected from a rabbit and subcutaneous tissue of the epithelium was removed by an enzymatic procedure so as to prepare a cell suspension containing stem cells. Then, the cell suspension was inoculated on amniotic membrane from which the epithelium was scraped and co-cultured with supporter cell (feeder cell), thereby inducing the differentiation. As a result, corneal epithelium-like cell layer (corneal epithelium-like sheet) in which a layered structure of the oral mucosal epithelial cells was formed could be successfully obtained. When the morphology of the obtained cell layer was observed, 5 to 6 layers of the cells were
laminated on the amniotic membrane and flat-shaped cells were present on the uppermost layer. Furthermore, the cell layer had a high transparency. The structure of the obtained cell layer was extremely similar to that of the corneal epithelium. On the other hand, the corneal epithelium-like cell layer was autologously transplanted on the eyes of a rabbit together with the amniotic membrane used as a substrate, and the survival thereof was observed. As a result, it was confirmed that the graft was survived and extended on the ocular surface, and the post-operative transparency was maintained. This shows that the use of oral mucosal epithelium as a cell source and the amniotic membrane as a substrate enables the corneal epithelium-like cell layer (corneal epithelium-like sheet), in which a layered structure of the oral mucosal epithelial cells is formed, to be constructed. Furthermore, this corneal epithelium-like layer can suitably be substituted for the corneal epithelium. The present invention was completed based on the findings and includes the following configurations.
[1] A corneal epithelium-like sheet, including a cell layer in which a layered structure of cells derived from oral mucosal epithelial cells is formed.
[2] The corneal epithelium-like sheet described in [1], wherein the cells of the outermost layer are not cornified.
[3] The corneal epithelium-like sheet described in [1] or [2], wherein the cells of the outermost layer are flat-shaped.
[4] The corneal epithelium-like sheet described in any of [1] to [3], wherein the corneal epithelium-like sheet has a barrier function.
[5] A corneal epithelium transplantation sheet, comprising a cell layer in which a layered structure of cells derived from oral mucosal epithelial cells is formed on a collagen layer.
[6] The corneal epithelium transplantation sheet described in [5], wherein the cells of the outermost layer of the cell layer are not cornified.
[7] The corneal epithelium transplantation sheet described in [5] or [6], wherein the cell of the outermost layer of the cell layer are flat-shaped.
[8] The corneal epithelium transplantation sheet described in any one of [5] to [7], wherein the collagen layer is derived from amniotic membrane.
[9] The corneal epithelium transplantation sheet described in any one of [5] to [7], wherein the collagen layer consists of amniotic membrane from which the epithelium has been removed.
[10] The corneal epithelium transplantation sheet described in any one of [5] to [9], wherein the corneal epithelium transplantation sheet has a barrier function.
[11] A method of constructing a corneal epithelium-like sheet, the method including:
   a) culturing oral mucosal epithelial cells on a collagen layer; and
   b) when the oral mucosal epithelial cells are proliferated and a layered structure of the cells is formed, bringing the outermost layer into contact with the air.
[12] The method of constructing a corneal epithelium-like sheet described in [11], wherein the step a) is carried out in coexistence of supporter cells.
[13] The method of constructing a corneal epithelium-like sheet described in [11], wherein the step a) is carried out in coexistence of supporter cells and in a state in which an isolation membrane with pore size through which the supporter cells cannot pass exists between the supporter cells and the collagen layer.
[14] The method of constructing a corneal epithelium-like sheet described in any one of [11] to [13], wherein the collagen layer is derived from amniotic membrane.
[15] The method of constructing a corneal epithelium-like sheet described in any one of [11] to [13], wherein the collagen layer consists of amniotic membrane from which the epithelium has been removed.
[16] A method of constructing a corneal epithelium-like sheet, including the steps of:
   inoculating supporter cells in a first container to form a supporter cell layer;
   setting a second container, which has a bottom face made of an isolation membrane with pore size through which the supporter cells cannot pass, in the first container so that the bottom face is located in a culture medium;
   forming a collagen layer on the bottom face of the second container;
   inoculating oral mucosal epithelial cells on the collagen layer;
   culturing the oral mucosal epithelial cells to form a layered structure of the cells; and
   bringing the outermost layer of the layered structure of the oral mucosal epithelial cells into contact with the air.
[17] The method of constructing the corneal epithelium-like sheet described in [16], wherein the collagen layer is derived from amniotic membrane.
[18] The method of constructing the corneal epithelium-like sheet described in [16], wherein the collagen layer consists of amniotic membrane from which the epithelium has been removed.

Note here that the "corneal epithelium-like sheet" herein is used as a term for meaning a cell layer having a feature similar to that of the corneal epithelium and capable of being substituted for the corneal epithelium.

Similarly, "corneal epithelium transplantation sheet" is used as a term for meaning a composition having a cell layer having a feature similar to that of the corneal epithelium and capable of being used in transplantation for reconstructing the corneal epithelium.

### BRIEF DESCRIPTION OF DRAWINGS

Fig. 1 shows images of the amniotic membranes taken by a scanning electron microscope. Fig. 1A shows an image of the surface of the amniotic membrane in the normal state (i.e., state in which treatment for scraping the epithelium is not carried out); and Fig. 1B shows the surface of the amniotic membrane after scraping the epithelium (in 0.02% EDTA solution). In Fig. 1A, polygonal-shaped amniotic epithelium is observed. On the other hand, in Fig. 1B, the amniotic epithelium is not observed and it is confirmed that epithelium is completely removed.

Fig. 2 is a cross-sectional view schematically showing a state of instruments, etc. when oral mucosal epithelial cells are cultured on amniotic membrane. In a culture dish 1, a culture insert 2 is disposed. On the bottom surface of the culture dish 1, a 3T3 cell layer 5 is formed. Furthermore, on the bottom surface of the culture insert 2, the amniotic membrane 3 is placed and oral mucosal epithelial cells 4 are cultured thereon. Reference numeral 6 denotes a culture medium.

Fig. 3 is an image taken by an optical microscope, showing a confluent state of the oral mucosal epithelium layer.

Fig. 4 shows a HE (hematoxylin-eosin) stained image of the oral mucosal epithelial cell formed on the amniotic membrane. It is observed that a layer including 5 to 6 layers of cells that are similar to corneal epithelium is formed.

Fig. 5 shows images of the oral mucosal epithelial cell layer (the oral mucosal epithelium sheet) on the amniotic membrane stained with antibodies raised against keratin 3 (K3) and keratin 12 (K12), respectively. Keratin 3 stainability is observed, whereas keratin 12 stainability is not. Note here that the stained part is shown by open.

Fig. 6 shows images of the oral mucosal epithelial cell layer (the oral mucosal epithelium sheet) on the amniotic membrane stained with antibodies raised against keratin 4 (K4) and keratin 13 (K13), respectively. Keratin 4 stainability and keratin 13 stainability are observed. Note here that the stained part is shown by open.

Fig. 7 shows images of the oral mucosal epithelial cell layer (the oral mucosal epithelium sheet) on the amniotic membrane stained with antibodies raised against keratin 1 and keratin 10, respectively. Neither keratin 1 stainability nor keratin 10 stainability is observed.

Fig. 8 is an image showing the surface of a rabbit eye in which the cornea and conjunctiva epithelium has been removed and which was stained with fluoresceine dyes. A part stained with fluoresceine dyes is shown by open. This images show that whole surface of the eye is stained with fluoresceine and epithelium has been completely removed.

Fig. 9 is an image showing the surface of a rabbit eye four weeks after the removal of the cornea and conjunctiva epithelium. This image shows that the transparency of the ocular surface is lost due to the invasion of the conjunctiva with cicatrical tissue from the peripheral part.

Fig. 10 is an image showing a state of the ocular surface after the transplantation of the corneal epithelium transplantation sheet. This image shows that the ocular surface (transplanted part) has transparency.

Fig. 11 shows an image stained with fluoresceine dyes 48 hours after the transplantation of the corneal epithelium transplantation sheet. A part stained with fluoresceine dyes is shown by open. This image shows that the ocular surface (transplant part) is not stained with fluoresceine dyes and the graft survived on the ocular surface. While it is found that the entire peripheral portion of the graft is stained with fluoresceine dyes.

Fig. 12 shows an image stained with fluoresceine dyes 10 days after transplantation of the corneal epithelium transplantation sheet. A part stained with fluoresceine dyes is shown by open. It is observed that a not-stained region extends and the graft remains on the ocular surface and further extends toward the periphery as compared with 48 hours after the transplantation.

Fig. 13 shows states of the ocular surface before operation (left image) and five months after the operation (right image) of a patient with prolonged epithelium defect in the acute stage of chemical trauma.

Fig. 14 shows states of the ocular surface before operation (left image) and two months after operation (right image) of a patient (cicatrical keratoconjunctive epithelium syndrome) at the chronic stage of chemical trauma.

Fig. 15 shows the states of the ocular surface before operation (left image) and one month after the operation (right image) of a patient with ocular cicatricial pemphigoid.

### BEST MODE FOR CARRYING OUT THE INVENTION

The first aspect of the present invention relates to a corneal epithelium-like sheet comprising a cell layer in which a layered structure of cells derived from oral mucosal epithelial cells is formed.

The "cell layer in which a layered structure of cells derived from oral mucosal epithelial cells is formed" herein denotes a cell layer formed by culturing cells of the oral mucosal epithelium part as a starting material to proliferate them, so that at least a part thereof is differentiated and layered. The cells for forming the cell layer are derived from, for example, oral mucosal epithelial cells such as cells of the oral inner marginal mucosa epithelium, cells of labial part, cells of palate part, cells of buccal part, and the like. The derivation of such cells can be confirmed by the indication that keratin 4 or keratin 13, which are specific to the mucosal epithelial cell, is expressed in the cells forming cell layer. Alternatively, it can also be confirmed by the indication that keratin 3 is expressed. This keratin 3 is known to be one of the keratins specific to cornea but it has been confirmed to be expressed also in the oral mucosal epithelial cell. Note here that it can be said that it is preferable to use oral mucosal epithelial cells for materials for producing compositions for corneal epithelium transplantation from the viewpoint in that this keratin 13 (cornea specific keratin) is expressed.

On the other hand, also by examining the expressions of genes specific to an oral mucosal epithelial cell, it can be confirmed that the cells forming the cell layer are derived from oral mucosal epithelial cells.

Preferably, the corneal epithelium-like sheet of the present invention has one or more than one of the following characters or properties. Particularly preferably, the corneal epithelium-like sheet of the present invention has all of the following characters or properties.
(1) The cells of the uppermost layer are not cornified. This is one of the features of corneal epithelium. When this feature is observed, the corneal epithelium-like sheet of the present invention is similar to corneal epithelium and is expected to exhibit the same function as that of corneal epithelium. Note here that "cornified" is also referred to as "keratinized", which represents the phenomenon in which keratin is generated in a cell and the cell organelle such as nucleus is lost. It can be confirmed whether or not the cells are cornified by, for example, the presence or absence of flatness or nucleus in a cell.
(2) The cells of the uppermost layer are flat-shaped. That is to say, an oral mucosal epithelial cell layer is configured by forming a layer of cells having flat shape on a layer of cells having approximately cuboidal shape. It is thought that when the uppermost layer is covered with flat-shaped cells, the tightness between cells is increased and a below-mentioned barrier function is attained. Also in corneal epithelium, cells in the uppermost layer are flat-shaped. When this feature is observed, the corneal epithelium-like sheet of the present invention is similar to corneal epithelium and is expected to exhibit the same function as that of corneal epithelium.
(3) A barrier function is provided. The barrier function means a function of preventing liquid, gas or the like from infiltrating from the surface or a function of preventing liquid from releasing via the surface layer. When such barrier function is provided, it is possible to maintain moisture (tear) on the surface after transplantation and to prevent more than necessary moisture from being released. Cornea can maintain moisture on the surface thereof as it has a barrier function, and thereby it resists blinking. Therefore, the barrier function is one of the most important features required for a material for cornea transplantation. When this feature is observed, the corneal epithelium-like sheet of the present invention is similar to corneal epithelium and is expected to exhibit the same function as that of corneal epithelium. Whether or not this barrier function is provided by examining the extent of infiltration of solution including an indicator such as Horseradish peroxidase.

This corneal epithelium-like sheet can be used as transplantation material (substitute for corneal epithelium) to a patient with damaged or failure cornea, etc. In transplantation, it is preferable that a graft is fixed to and allowed to survive by fixing it to the surrounding tissue with a surgical suture. Furthermore, it is preferable that after transplantation, the surface of the transplanted part is protected by temporarily being covered with a therapeutic contact lens.

The second aspect of the present invention provides a corneal epithelium transplantation sheet having the following configurations. That is to say, it provides a corneal epithelium transplantation sheet, comprising a cell layer in which a layered structure of cells derived from oral mucosal epithelial cells is formed on a collagen layer. In other words, it provides a corneal epithelium transplantation sheet in which the above-mentioned corneal epithelium-like sheet is formed on a collagen layer. It is preferable that the collagen layer herein is derived from amniotic membrane. It is further preferable to use the amniotic membrane from which the epithelium has been removed by scraping procedure, etc. Whether or not the collagen layer is made of the amniotic membrane from which the epithelium has been removed can be confirmed by examining that a cell of amniotic epithelium layer is not contained in the collagen layer. Note here that it is preferable that human amniotic membrane is used as the amniotic membrane.

The corneal epithelium transplantation sheet can be used as transplantation material (substitution for corneal epithelium) to patients with damaged or failure cornea, etc. In this case, the sheet is transplanted to the corneal epithelium defective part so that the collagen layer side is located at the side of the eyeball. In transplantation, it is preferable to promote survival of a graft by fixing it to the surrounding tissue with a surgical suture. Furthermore, it is preferable that after transplantation, the surface of the transplanted part is protected by temporarily being covered with a therapeutic contact lens.

The above-mentioned corneal epithelium-like sheet and corneal epithelium transplantation sheet can be constructed by the following methods.

The third aspect of the present invention relates to a method for constructing a corneal epithelium-like sheet and includes the following steps of:
a) culturing oral mucosal epithelial cells on a collagen layer; and
b) when the oral mucosal epithelial cells are proliferated and a layered structure of the cells is formed, bringing the outermost layer into contact with the air.

Herein, the kinds of collagen as a raw material of the collagen layer is not particularly limited, and type I collagen, type III collagen and type IV collagen and the like can be used. A plurality of collagens can be used in combination thereof. Such collagens can be extracted and purified from the connective tissue of the skin and cartilage, etc. of animals such as swine, bovine, sheep, and the like, by an acid solubilization method, alkali solubilization method, and oxygen solubilization method, and the like. Note here that for the purpose of lowering the antigenicity, a so-called atherocollagen obtained by removing telopeptide by the treatment with the use of catabolic enzyme such as pepsin, trypsin, etc.

It is preferable that as the collagen layer, one derived from amniotic membrane, particularly derived from human amniotic membrane is used. Herein, the collagen layer is "derived from amniotic membrane" herein broadly means that the collagen layer is obtained by using amniotic membrane as a starting material. Human amniotic membrane is a membrane covering the outermost layer of the uterus and the placenta, and a basal membrane and an epithelium layer are formed on parenchymal tissue that is rich in collagen. Human amniotic membrane can be collected by, for example, human embryonic membrane, placenta, etc. obtained at the time of afterbirth at delivery. Specifically, the human amniotic membrane can be prepared by treating and purifying the integrated material including human embryonic membrane, placenta, and umbilical cord obtained right after delivery. The method of treating and purifying can employ a method described in JP 5(1993)-5698A, etc. That is to say, amniotic membrane is detached from the embryonic membrane obtained at delivery and remaining tissue is removed by a physical treatment such as ultrasonic cleansing and an enzyme treatment, and the like. Then, appropriate cleaning process is carried out and thus human amniotic membrane can be prepared.

The thus prepared human amniotic membrane can be cryopreserved before use. The human amniotic membrane can be frozen in a liquid mixing equal volume ratio of DMEM (Dulbecco's modified Eagle's medium) and glycerol at, for example, -80°C. By the cryopreservation, not only the improvement in operation but also reduction of the antigenicity can be expected.

Intact amniotic membrane is may be used as a collagen layer but it is preferable that amniotic membrane from which the epithelium has been removed by a scraping treatment, etc. is used. For example, after thawing, cryopreserved human amniotic membrane is subjected to a treatment with EDTA or proteolytic enzyme so as to loosen the adhesion between cells and then the epithelium is scraped by using a cell scraper, etc. Thus, human amniotic membrane from which the epithelium has been removed can be prepared.

On the collagen layer, oral mucosal epithelial cells are cultured. It is suggested that oral mucosal epithelium has stem cells and it is thought to easily induce differentiation of them to cells forming an epithelium-like cell layer. Furthermore, the use of oral mucosal epithelial cells has the following advantages: they can be collected easily; a large number of cells can be collected; and when a patient with bilateral-eye disease is treated, transplantation material derived from the autologous cells can be prepared. In particular, with the advantage that a patient from which corneal epithelium cells cannot be collected, transplantation materials derived from autologous cells can be used, it is expected that the clinically important problem about immunological rejection can be significantly solved.

As the oral mucosal epithelial cell, a cell existing in the dental root part (a cell of the oral inner marginal mucosa epithelium), a cell of labial part, a cell of palate part, a cell of buccal part, and the like, can be used. Among them, it is particularly preferable to use a cell of oral inner marginal mucosa epithelium because it has a high proliferation ability and low antigenicity. The oral mucosal epithelial cells can be collected by ablating a site where targeted cells exists by using a scalpel or by scraping it out. Oral inner marginal mucosa epithelium can be collected from the oral inner marginal mucosa epithelium which was separated from enamel cement transition portion. Note here that in order to remove impurities such as connective tissue, preferably a treatment with enzyme such as Dipase or trypsin, etc. , filtration treatment are carried out.

Oral mucosal epithelial cells collected from an oral cavity of an individual other than a patient to whom an corneal epithelium-like sheet constructed according to the present invention is transplanted may be used. However, when considering the immunological rejection, preferably the oral mucosal epithelial cells from a patient him/herself is collected and cultured.

The thus collected oral mucosal epithelial cells are cultured on a collagen layer. For example, a suspension liquid of oral mucosal epithelial cells are prepared by the above-mentioned method, and inoculated on the collagen layer and cultured under an appropriate culture conditions. When human amniotic membrane from which the epithelium has been removed is used as the collagen layer, the oral mucosal epithelial cells are preferably inoculated on the side of the collagen layer with the side where the epithelium is removed and exposed (i.e., the side of the basal membrane). It is thought that this side is rich in type IV collagens and the inoculated oral mucosal epithelial cells can be proliferated and layered well.

The oral mucosal epithelial cells can be inoculated on the collagen layer so that, for example, the cell density becomes about 1×10³ cells/cm² or more, preferably in the range from about 1×10³ cells/cm² to about 1×10⁷ cells/cm², and further preferably in the range from about 1×10⁴ cells/cm² to about 1×10⁶ cells/cm².

The oral mucosal epithelial cells are preferably cultured in the presence of supporter cells. The supporter cell is referred to as also a feeder cell and supplies growth factor, etc. with culture medium. When the oral mucosal epithelial cells are cultured in the coexistence of supporter cells, the proliferation efficiency of the oral mucosal epithelial cells is improved. As the supporter cells, for example, a 3T3 cell (Swiss mouse 3T3 cell, mouse NIH3T3 cell, 3T3J2 cell, etc.) and the like may be used. Among them, it is preferable to use a mouse NIH3T3 cell as a supporter cell from the viewpoint of proliferation efficiency, ease in handling, etc.

It is preferable that the supporter cells are inactivated by using mitomycin C, etc. It is advantageous because the inhibition of the proliferation of the oral mucosal epithelial cells due to the proliferation of the supporter cells themselves is prevented, and the proliferation efficiency of the oral mucosal epithelial cells is enhanced. Such inactivation can also be carried out by a radiation treatment, etc.

When the oral mucosal epithelial cells are cultured in the coexistence of supporter cells, it is preferable that an isolation membrane having a pore size through which the supporter cells cannot path is provided between the supporter cells and the collagen layer. The use of the separating membrane makes it possible to prevent the supporter cells from entering the side of the collagen layer (i.e. the side of oral mucosal epithelial cells) at the time of cultivation. As a result, the supporter cells may not be mixed in the finally obtained corneal epithelium-like sheet. This means that a corneal epithelium-like sheet being free from problem of immunological rejection by the supporter cells can be constructed. This is clinically significant so much.

As the isolation membrane, an isolating membrane having a pore size through which the supporter cells cannot path can be used by selecting the known membrane appropriately. For example, a membrane having a pore size of about 0.4 µm to 3.0 µm made of polycarbonate can be used. A material of the isolation membrane is not particularly limited. Besides polycarbonate, polyester and the like may be used. Such isolation membranes are on the market and readily available.

An example of the culture method using an isolation membrane may include the following method. Firstly, inactivated supporter cells are inoculated and cultured on a container such as a dish (a first container), thereby forming a layer of supporter cells on the surface of the container. Then, a second container, which has a bottom face made of an isolation membrane, is set in the first container so that the bottom face of the second container is located in a culture medium. Then, on the collagen layer, oral mucosal epithelial cells are inoculated and cultured.

On the bottom surface of the second container, a collagen layer is previously formed (for example, on the bottom surface of the second container, the amniotic membrane from which the epithelium has been removed is placed. In this state, drying process may be carried out). This second container is set in the first container in which supporter cells are inoculated, and then on the collagen layer, oral mucosal epithelial cells may be inoculated and cultured.

The cell density of the supporter cells may be, for example, about 1×10² cells/cm² or more, preferably in the range from about 1×10² cells/cm² to about 1×10⁷ cells/cm², and further preferably in the range from about 1×10³ cells/cm² to about 1×10⁵ cells/cm². As to the ratio with respect to the number of the oral mucosal epithelial cells, culture may be carried out under the conditions in which the supporter cells to be used may be, for example, 1/10³ times to 1×10² times, and preferably 1/10² times to 1 time as the number of the oral mucosal epithelial cells. When the number of the supporter cells is small, the proliferation rate of the oral mucosal epithelial cells is lowered; and when it is too small, excellent layered structure of the oral mucosal epithelial cells cannot be obtained. On the other hand, it is not preferable that the number of the supporter cells is too large, because the proliferation rate of the oral mucosal epithelial cells is lowered.

The culture medium used for culturing the oral mucosal epithelial cells is not particularly limited as long as the cells can be proliferated and a layered structured of the cells can be formed. For example, a medium, in which DMEM (Dulbecco's modified Eagle's medium) that is generally used for growing epithelial cells and Ham's F12 medium are mixed with each other at the predetermined ratio, and FBS, growth factor, antibiotics, and the like are added, may be used. Specific examples include a mixing medium of DMEM and Ham's F12 medium (mixing volume ratio of 1 : 1) to which FBS (10%), insulin (5 mg/ml), cholera toxin (0.1 nM), epithelial cell growth factor (EGF) (10 ng/ml) and penicillin-streptomycin (50 IU/ml) are added. Furthermore, a mixing medium of DMEM and Ham's F12 medium to which triiodothyronine (e.g. 2 nM), glutamine (e.g. 4 mM), transferrin (e.g. 5 mg/ml), adenine (e.g. 0.18 mM), and/or hydrocortisone (e.g., 0.4 mg/ml) are further added, may be used.

In step a), the oral mucosal epithelial cells are proliferated on the collagen layer and layered structure of cells is formed. Then, a step (step (b)) of bringing the surface layer of the superimposed cell layer into contact with the air is carried out. Note here that this step herein is referred to as also Air lifting. This step (b) is carried out for differentiation of cells forming the cell layer and inducing the barrier function.

This step can be carried out by lowering the surface of the culture medium by temporarily removing a part of the culture medium by using a dropper, a pipette, and the like, thereby temporarily exposing the outermost layer of the oral mucosal epithelial cell layer to the outside of the culture medium. Alternatively, this step can be carried out by lifting up the oral mucosal epithelial cell layer together with the collagen layer, thereby temporarily exposing the outermost layer from the culture medium surface. Furthermore, by using the tube etc., the air may be fed into the culture medium so as to bring the uppermost layer of the oral mucosal epithelial cell into contact with the air. From the viewpoint of the ease in operation, it is preferable that by lowering the surface of the culture medium, thereby exposing the outermost layer of the oral mucosal epithelial cell layer to the outside.

The period when this step (b) is done, that is, the period of time when the uppermost layer of the layer of layered structure of cells is brought into contact with the air differs depending upon the state of the cells or culture conditions, etc. but it may be for example 3 days to 3 weeks, preferably 5 days to 2 weeks, and further preferably for about one week.

According to the above-mentioned method of the present invention, on the collagen layer, a corneal epithelium-like cell layer (corneal epithelium-like sheet), in which the oral mucosal epithelial cells are layered, is formed. This corneal epithelium-like sheet together with the collagen layer that is used as a substrate of the oral mucosal epithelial cells can be used as a transplantation material (substitute for the corneal epithelium) for patients with injured or defective cornea, etc. In this case, the corneal epithelium-like sheet is transplanted to the corneal epithelium defective part so that the collagen layer is located to the side of the eyeball. In transplantation, it is preferable to promote survival of the graft by fixing it to the surrounding tissue with a surgical suture. Furthermore, it is preferable that after transplantation, the surface of the transplanted part is protected by temporarily being covered with a therapeutic contact lens.

Note here that a graft from which a part or all of the collagen layer is removed (that is, only the corneal epithelium-like sheet) may be used. The collagen layer can be removed by appropriately combining a chemical treatment with EDTA, etc., an enzymatic treatment by proteolytic enzyme, etc., and a physical treatment such as scraping by using forceps.

According to the third aspect of the present invention, an corneal epithelium-like cell layer (corneal epithelium-like sheet) in which oral mucosal epithelial cells are layered, is constructed. However, it is thought that the method of the present invention can provide a transplantation material (an corneal epithelium transplantation sheet) for cornea, in which the cell layers are formed on the collagen layer. That is to say, the method of the present invention can be used as a method of constructing the corneal epithelium transplantation sheet having such a structure.

### <Example 1>

### Evaluation of corneal epithelium-like sheet using rabbit [Preparation of amniotic membrane]

After giving a pregnant woman who does not have a systemic complication and would undergo Caesarean section sufficient informed consent together with an obstetrician in advance, the amniotic membrane was obtained during the Caesarean section in the operation room. The operation was carried out cleanly. In accordance with the operation work, the operators washed hands, and then wore a special gown. Before delivery, a clean vat for obtaining amniotic membrane and physiologic saline for washing were prepared. After delivery, the placenta tissue was transferred to the vat and the amniotic membrane tissue was manually removed from the placenta. A portion where amniotic membrane and placenta were strongly adhered to each other was separated wit scissors.

### [Treatment of amniotic membrane]

Treatment process of amniotic membrane included: (1) washing, (2) trimming, and (3) storing sequentially in this order. Throughout all the processes, operation is desired to be carried out in a clean draft. For all containers and instruments for use, those sterilized were used, and for dishes, etc. sterilized disposable ones were used. The obtained amniotic membrane was washed for removing blood component attached thereto and further washed in a sufficient amount of physiological saline (0.005% ofloxacin was added). Then, the amniotic membrane was transferred to a phosphate buffer solution (PBS) in a dish and cut and divided into the size of about 4×3 cm with scissors. The divided pieces of amniotic membrane were stored in several dishes filled with a stock solution, and thereafter amniotic membranes in good condition were selected among them.

### [Storage of amniotic membrane]

1 cc each of stock solution was placed in 2 cc sterilized cryotube and one sheet each of the amniotic membrane, which had been obtained, washed and selected, was placed and labeled, then stored in a refrigerator at -80°C. For the stock solution, 50% sterilized glycerol in DMEM (Dulbecco's Modified Eagle Medium: GIBCOBRL) was used. The expiration date for use of stored the amniotic membrane was determined at 3 months and expired amniotic membrane was disposed of by incineration.

### [Treatment of amniotic epithelium]

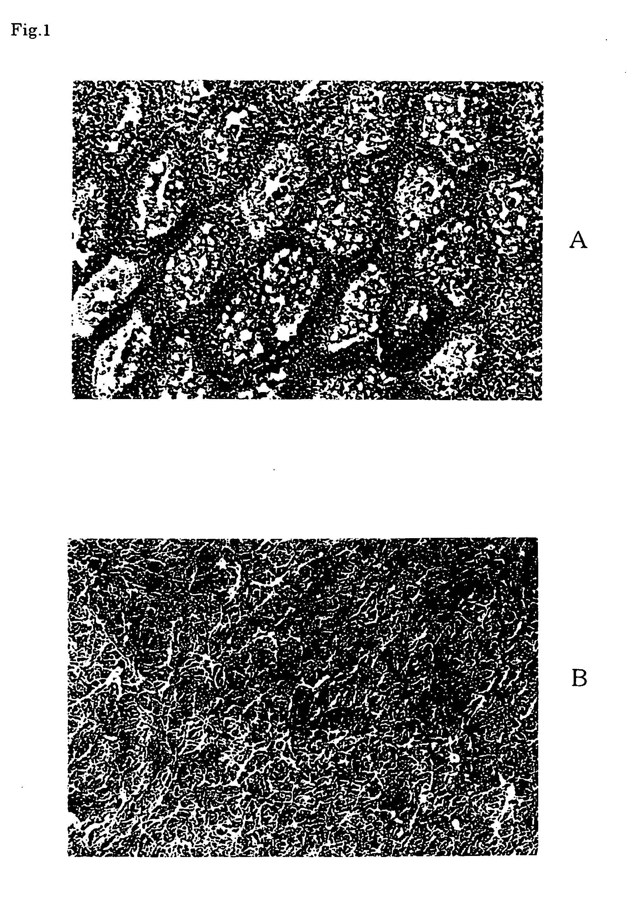

The amniotic membrane was subjected to treatment for removing an epithelium and then used for culture. First of all, the amniotic membrane stored at -80° C was thawed at room temperature, and then well washed in sterilized phosphate buffer solution (PBS) in the dish. After washing, the amniotic membrane was stored in a 0.02% EDTA solution (Nacalai tesque) at 37° C for 2 hours, and then the epithelium was mechanically scraped off by using a cell scraper (Nunc, USA) and used as a substrate for culture. Note here that, it was confirmed that one layer of the amniotic epithelium was completely scraped by this treatment process by the optical microscope and electron microscope (scanning electron microscope) operations (Fig. 1). Note here that Fig. 1 shows images of the amniotic membrane by the scanning electron microscope. Fig. 1A shows an image of the surface of the amniotic membrane in the normal state (i.e., state in which an epithelium procedure is not carried out); and Fig. 1B shows the surface of the amniotic membrane after scraping the epithelium (in 0.02% EDTA solution).

### [Collection of oral mucosal epithelial cells]

In 6-week old Japanese white rabbit, tooth was pulled out. Then, the oral mucosal epithelium was carefully separated from the enamel cement transition portion. Note here that a series of operations were carried out by using sterilized instruments as antiseptically as possible.

The obtained oral mucosal epithelium was immersed twice in a phosphate buffer solution (PBS) containing 50 IU/ml penicillin streptomycin and Gentacin for 30 minutes under the condition of room temperature. Thereafter, the tissue was immersed in a phosphate buffer solution (PBS) containing 1.2U Dispase (Nacalai tesque) for 30 minutes at 37° C and immersed and treated in 0.05% trypsin-EDTA solution (GBCOBRL) for 30 minutes so as to separate cells. An enzyme activity was stopped by immersing in DMEM containing 10% fetal bovine serum (FBS). Thereafter, excess tissues were removed by using a 60 µm cell filter so as to isolate the oral mucosal epithelial cells (oral inner margin epithelial cells).

### [Preparation of co-cultured cells]

As the co-culture cells (support cells), NIH-3T3 cells (hereinafter, referred to as "3T3 cell") were used. 3T3 cell which had been cultured in advance and become confluent in 75F flask (BD product of Falcon) were immersed in 0.05% mitomycin C solution for two hours so as to suppress the proliferation activity. Sequentially, they were washed with a phosphate buffer solution (PBS) several times so as to remove mitomycin C, followed by treating with 0.05% trypsin-EDTA solution (PBS) so as to prepare a 3T3 suspension.

### [Formation of oral mucosal epithelium sheet on amniotic membrane]

By using human amniotic membrane from which an epithelium was scraped as a substrate, the oral mucosal epithelial cells were co-cultured with 3T3 cells that were subjected to the above-mentioned treatment by the following procedure. For culturing instruments, a 6-well culture dish (Corning, NY) and a culture insert (a container for inserting culture) (polycarbonate, average pore size: 3.0 µm, Corning NY) were used.

First of all, the 3T3 suspension was inoculated on the culture dish so that the cell density was about 1×10⁴ cells/cm² and cultured under conditions at 37° C and in 5%CO₂. Furthermore, the amniotic membrane substrate was allowed to stand still to be fixed on the culture insert with the side of the scraped epithelium upward, and dried for 10 minutes at room temperature. Thereafter, on the culture insert to which the amniotic membrane was attached, the suspension containing an oral mucosal epithelial cell was inoculated so that the cell density was about 1×10⁵ cells/cm².

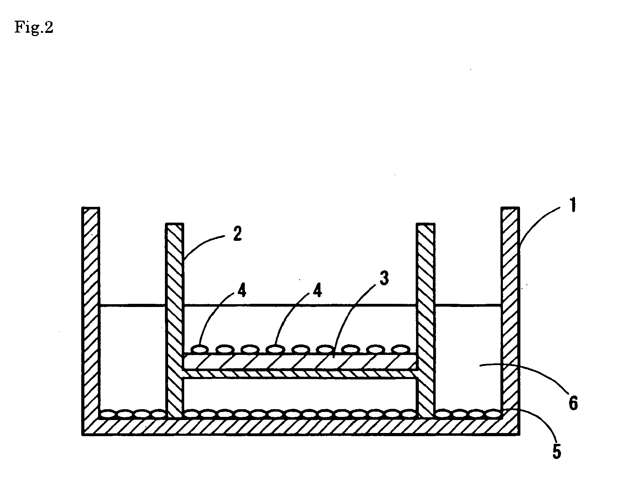

After the above-mentioned operation, as shown in Fig. 2, the culture insert was disposed in the culture dish and the 3T3 cells and the oral mucosal epithelial cells were cultured in the same culture medium. Note here that Fig. 2 is a cross-sectional view schematically showing a state during culturing. In the culture dish 1, the culture insert 2 is placed and on the bottom surface of the culture dish 1, the 3T3 cell layer 5 is formed. Furthermore, on the bottom surface of the culture insert 2, the amniotic membrane 3 is placed, and the oral mucosal epithelial cells 4 are cultured thereon. Reference numeral 6 denotes a culture medium.

As the culture medium, a DMEM / Ham's F12 mixture medium (mixing volume ratio: 1:1) including 10% FBS, insulin (5 mg/ml), cholera toxin (0.1 nM), penicillin-streptomycin (50 IU/ml) and human recombinant epithelial cell growing factor (10 ng/ml) was used.

The culture was carried out in the above-mentioned medium for two weeks (Submerge). Thereafter, for inducing the mucosal epithelium, by a so-called Air-lifting method, culture was carried out for a week. The Air-lifting method is a method of lifting the liquid surface of the culture medium to the surface of the oral mucosal epithelial cell layer formed on the amniotic membrane to bring the cell layer into contact with the air. During submerging, the culture medium was replaced with new one every other day and after carrying out the Air-lifting method, the culture medium was replaced with new one every day.

### [Verification of the physiological property of oral mucosal epithelium sheet on amniotic membrane]

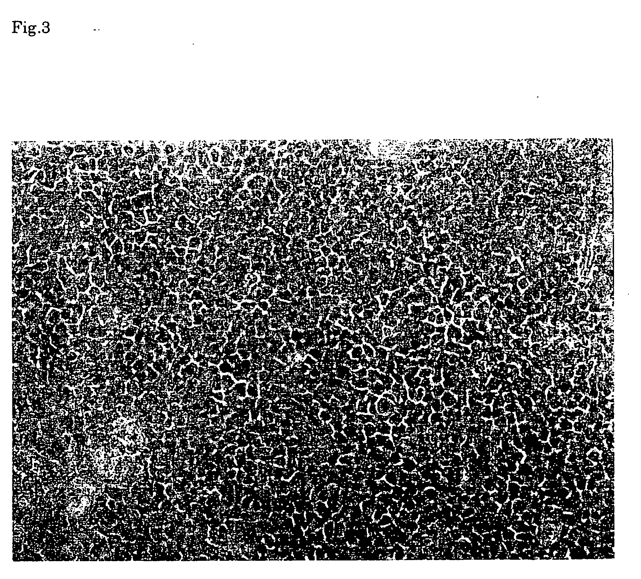

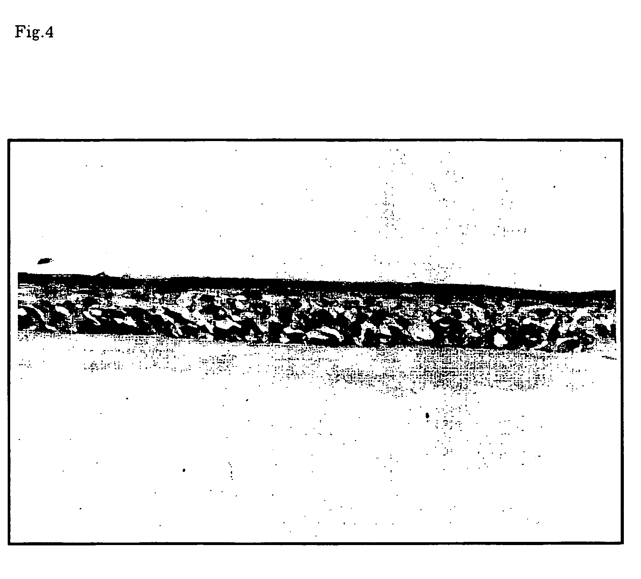

The oral mucosal epithelial cell layer culture as mentioned above was cultured for about 20 days, thereby forming an epithelium layer similar to corneal epithelium including 5 to 6 multi-differentiated and layer-structured layers (hereinafter, also referred to as "corneal epithelium-like sheet") (see Figs. 3 and 4). At the basal side (the side of the amniotic membrane) of this epithelium layer, a group of relatively cuboidal-shaped cells similar to the basal cell existed. Furthermore, it was confirmed that the cells of the outermost layer had a flat shape but included a nucleus and that the surface thereof was not cornified unlike the skin. As mentioned above, the optical microscope observation showed that the epithelium layer similar to cornea (corneal epithelium-like sheet) was formed on amniotic membrane.

Then, in order to confirm the physiological property of the corneal epithelium-like sheet, immunostaining was carried out. After an corneal epithelium-like sheet was prepared, it was cut into an appropriate size and frozen and embedded in an OCT compound. Then, the resultant compound was sliced with a cryostat to prepare slide sections. In immunostaining, the consideration on keratin, that is, respective cytoskeleton protein was carried out. That is to say, keratin 3/12 specific to the cornea, keratin 4/13 specific to mucosa and keratin 1/10 specific epidermis were considered. The method will be described below. A slide section was washed with a phosphate buffer solution (PBS) and then blocking with 1% FBS was carried out to suppress the non-specific antibody reaction. Thereafter, an antibody against each keratin (primary antibody) was reacted at room temperature for one hour. After reaction, the slide section was washed with PBS containing triton-X for 15 minutes three times, followed by reacting with fluorescence labeling antibody (secondary antibody) at room temperature for one hour. After reaction, the slide section was washed with a phosphate buffer solution (PBS) for 15 minutes three times and sealed, followed by observing the tissue with a confocal microscope.

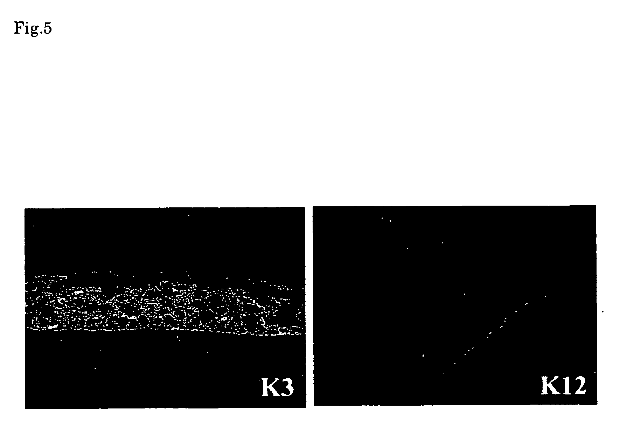

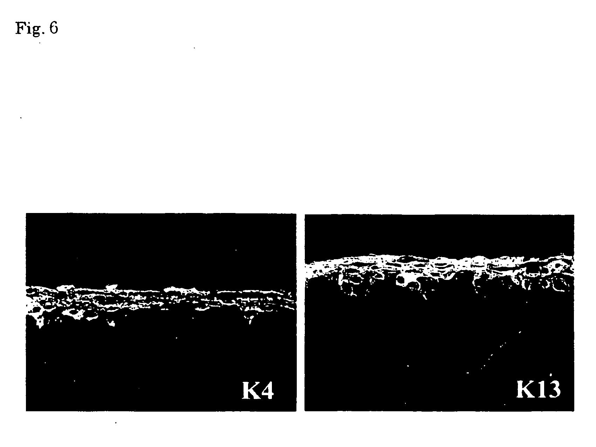

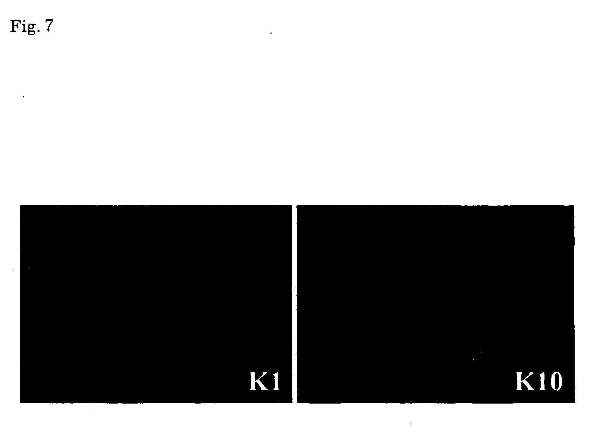

The antibody reactions of the respective keratins with respect to the corneal epithelium-like sheet are described. Firstly, for the keratin 3 specific to cornea, stainability was observed (see Figure 5, left image). Since keratin 3 stainability observed in oral mucosa in vivo, it was thought that the property was maintained also under the culture conditions. On the other hand, for the keratin 12, stainability was not observed (see Fig. 5, right image). Similarly, for the keratin 4 and 13 specific to mucosa, the stainability was also observed respectively (see Fig. 6). However, for keratin 1/10 that is an epidermal cornification keratin, the stainability was not observed (see Fig. 7). From the above-mentioned results, as to the physiological property of the corneal epithelium-like sheet, from the aspect of the cytoskeleton, differentiation does not occur in the direction of the cornification unlike the epidermis. It was confirmed that the property as non-cornified mucosal epithelium was maintained while maintaining a part of the keratin (keratin 3) specific to cornea was maintained.

### [Autologous transplantation of oral mucosal epithelium sheet]

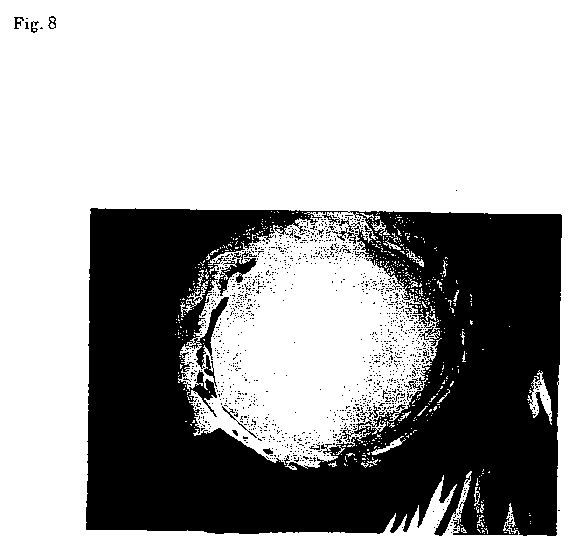

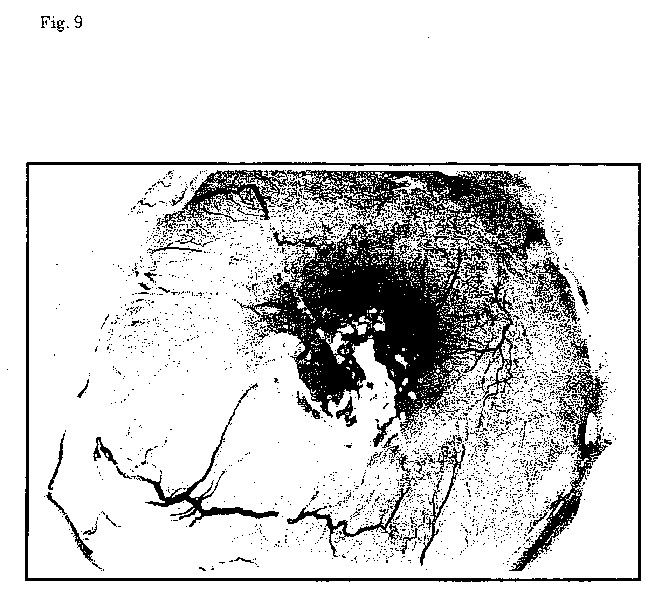

Next, experiment of autologous transplant using the corneal epithelium-like sheet was carried out. By the above-mentioned method, the oral mucosal epithelial cells were collected from a 6-week old Japanese white rabbit to construct a transplantation sheet in which a corneal epithelium-like sheet was formed on amniotic membrane (hereinafter, referred to also as "corneal epithelium transplantation sheet"). Meanwhile, to the white rabbit from which the oral mucosal epithelial cell was collected, all the cornea and conjunctival epithelium having a thickness of 100 µm were removed from 4-mm outside of the limbus by using a crescent knife. By this operation, since the epithelial cells containing corneal epithelium stem cells are lost, artificial exhaustion of the ocular surface stem cells was thought to be reappeared. After this operation, it was confirmed by the fluoresceine staining that epithelium containing corneal epithelium cells did not remain (see Fig. 8). Note here that fluoresceine staining test was carried out as follows: that is, a fluoresceine test paper impregnated with instillation such as antibiotics was applied directly on the ocular surface, followed by allowing the eye to blink a few times, and then the fluoresceine staining on the ocular surface was observed. If the corneal epithelium is left, due to the tight intercellular adhesiveness structure, the fluoresceine dye is not saturated and fluoresceine-staining is not observed. 4 weeks later, the ocular surface of the rabbit was covered with the remaining conjunctival epithelium and did not maintain the transparency (see Fig. 9).

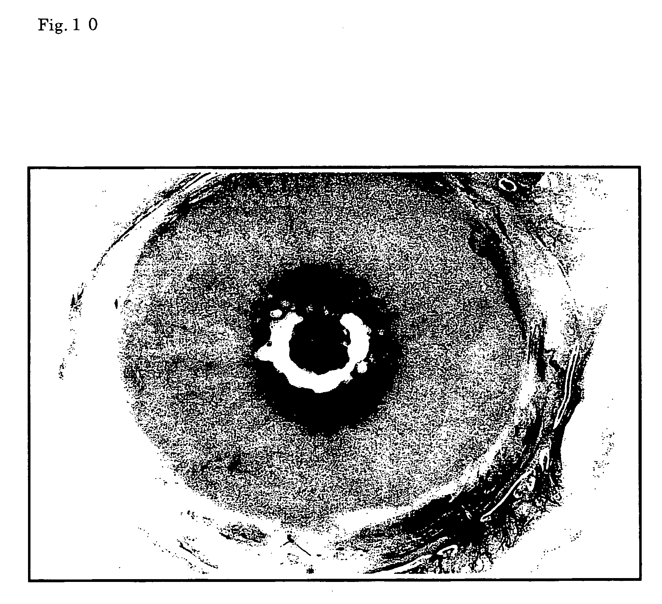

Then, to the eye which was covered with the conjunctival epithelium and lost the transparency, after the conjunctival tissue on the ocular surface was removed, the above-mentioned corneal epithelium transplation sheet is transplanted into the region rather inner from the limbus. In transplantation, by using 10-0 nylon fiber was used to stitch the sheet to the peripheral tissue. After transplantation, on the graft, a therapeutic contact lens was placed. After the operation, antibiotics and steroid ophthalmic ointment were applied twice a day. At the time of transplantation, the ocular surface had a transparency that is the same as that of the corneal epithelium transplantation sheet before transplantation (see Fig. 10).

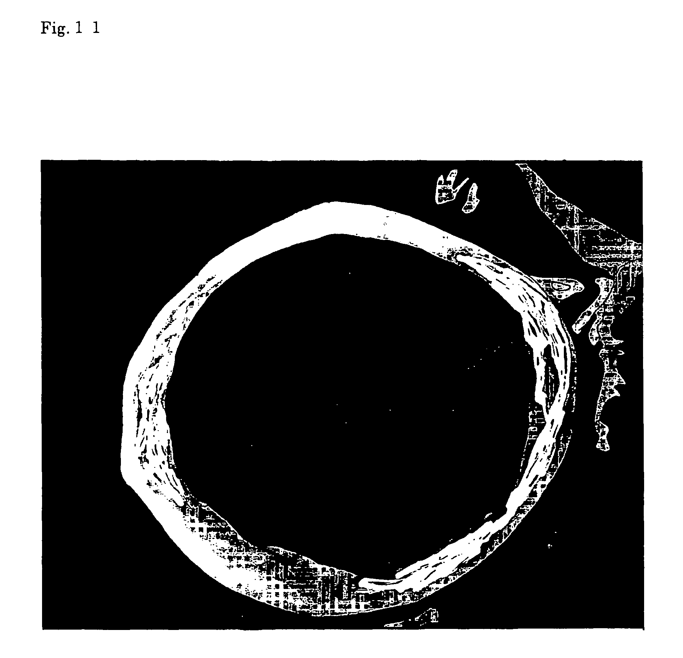

48 hours after the operation and 10 days after the operation, the ocular surface to which the transplantation was carried out was observed. At 48 hours had passed after the operation, it was confirmed that the transplanted corneal epithelium transplantation sheet maintained transparency. Furthermore, it was confirmed by fluoresceine staining that the transplanted corneal epithelium transplantation sheet remained on the ocular surface without being damaged (see Fig. 11). Furthermore, since the graft (corneal epithelium transplantation sheet) did not show the stainability with fluoresceine, it was confirmed that the corneal epithelium transplantation sheet (corneal epithelium-like sheet) of the present invention had a barrier function similar to the corneal epithelium. Furthermore, since by the fluoresceine staining, stainability with fluoresceine was confirmed over the entire periphery of the graft, therefore it was confirmed that the tissue existing in the transplanted part was not contamination of the remaining conjunctival epithelium.

Note here that since cells of the corneal epithelium are tightly adhered to each other, the fluoresceine dye does not invade from the surface and stainability with fluoresceine is not observed in straining with fluoresceine. On the other hand, when the adhesion between cells becomes loosen or the barrier function is damaged by exfoliation of the cell itself, invasion of the fluoresceine dyes occur, and the tissues are stained. Therefore, by examining the stainability of fluoresceine staining was examined, it can be confirmed whether or not the transplanted corneal epithelium-like sheet had the barrier function similar to corneal epithelium.

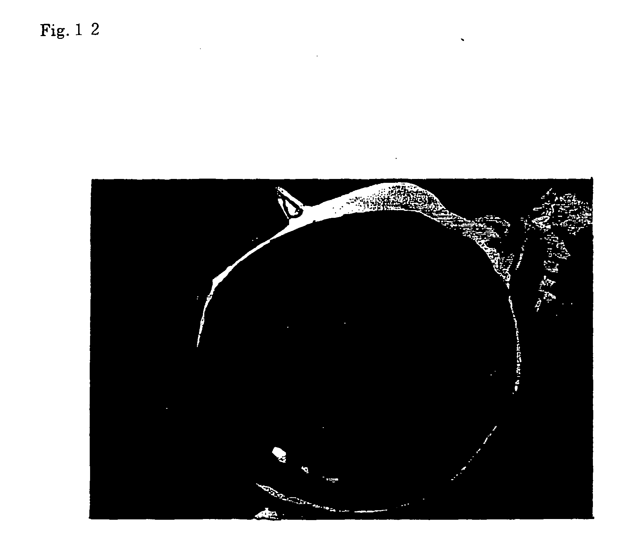

When 10 days had passed after the transplantation, the ocular surface was observed similarly to the above. The transplanted corneal epithelium transplantation sheet remained on the ocular surface. Furthermore, it was observed from the fluoresceine staining that the corneal epithelium transplantation sheet extends to the periphery more than the state in which 48 hours after the transplantation (see Fig. 12). It was confirmed that the transplant did not exhibit the stainability of fluoresceine and that a barrier function necessary to corneal epithelium was maintained. Also the transparency was maintained.

As mentioned above, it was confirmed that the corneal epithelium-like sheet obtained by culturing on the amniotic membrane survived on the ocular surface and extended on the ocular surface and maintained transparency for a long time after the operation. That is to say, the corneal epithelium-like sheet constructed by the above-mentioned method functions well as a substitute for corneal epithelium, and a sheet-like composition (corneal epithelium transplantation sheet) including the corneal epithelium-like sheet formed on the amniotic membrane can be suitably used as a transplant material for reconstructing the ocular surface in the case where the cornea was injured and damaged.

### <Example 2>

### Evaluation of corneal epithelium-like sheet in human

Then, the effect of the case where the corneal epithelium-like sheet was applied for a human was confirmed. Subjects to be transplanted included (1) patients with a prolonged epithelium defect in an acute stage of a chemical trauma; (2) patients with haze due to the invasion of cicatrical tissue in a chronic stage of a chemical trauma; and (3) patients with ocular cicatricial pemphigoid. Oral mucosal epithelial cells were collected from each patient by the same method as in the above-mentioned Example 1. Subsequently, the oral mucosal epithelial cells were co-incubated with 3T3 cells by using human amniotic membrane from which the epithelium had been scraped as a substrate to obtain a corneal epithelium transplantation sheet in which an oral mucosal epithelial cell layer was formed on the human amniotic membrane. Note here that a method of preparing the human amniotic membrane from which the epithelium had been scraped and the conditions for co-culturing with 3T3 were the same as in Example 1. The thus prepared corneal epithelium transparent sheet was transplanted on the patients whose cells were used for preparation the sheet (autologous transplant) and then the effect thereof was evaluated. The transplanting method was carried out by the same method as in Example 1. However, for patients with ocular cicatricial pemphigoid, a cicatrical tissue in the anterior part was removed so as to release the adhesion, then the corneal epithelium-like sheet was transplanted in the anterior one-third part and at the same time an operation for cataract was carried out to insert the intraocular lens.

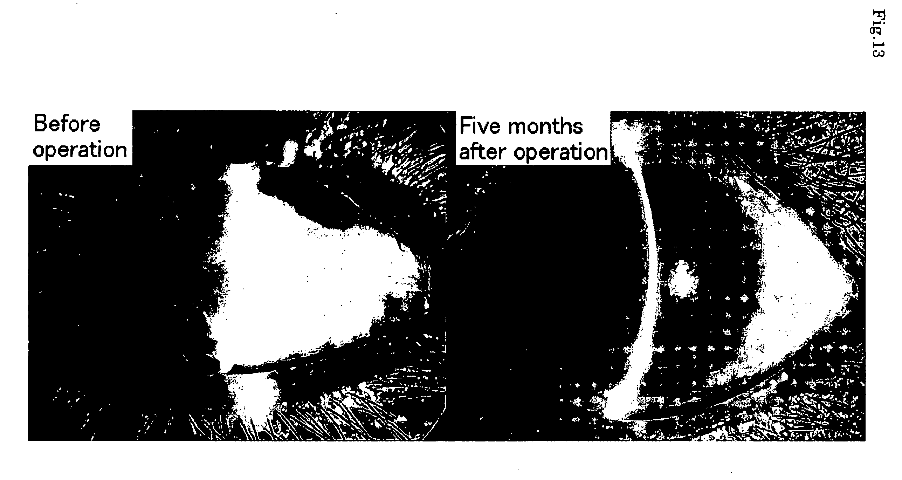

Fig. 13 shows the states of the ocular surface before the operation (left image) and five months after the operation (right image) of a patient with prolonged epithelium defect in an acute stage of chemical trauma. Fig. 13 shows that five mounts after the operation, the ocular surface is successfully reconstructed. The visual acuity of this patient was expected to be about a hand motion level due to the invasion of cicatrical tissue if the transplantation operation was not carried out. At the present, the visual acuity is recovered to 0.5, and the state of the epithelium is stable. Furthermore, since the transplantation was carried out by using the autologous transplant, there is no fear of immunological rejection, so that postoperative care is much easier as compared with the case in the transplantation operation.

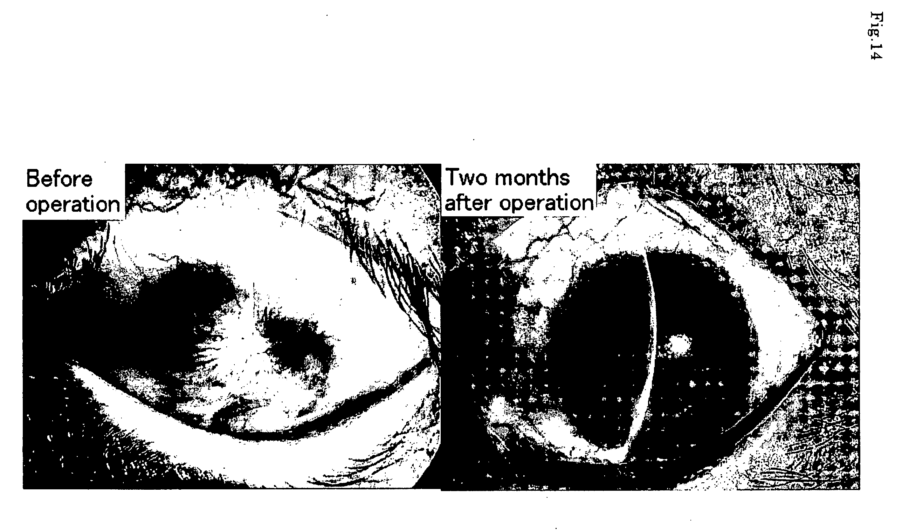

Fig. 14 shows the states of the ocular surface before the operation (left image) and two months after the operation (right image) of a patient with cicatric keratoconjunctive epithelium disease) in a chronic stage of chemical trauma. Before the operation, the condition of this patient was too serious to obtain the findings of intraocular condition. However, adhesion could be released by the transplantation operation, so that the crystalline lens could be seen clearly. The visual acuity was improved from the level of light perception to a level of counting fingers. The operation was carried out for determining the following operation plan, however the original purpose for carrying out an intraocular observation was achieved and furthermore, significant improvement in terms of beautiful appearance was obtained.

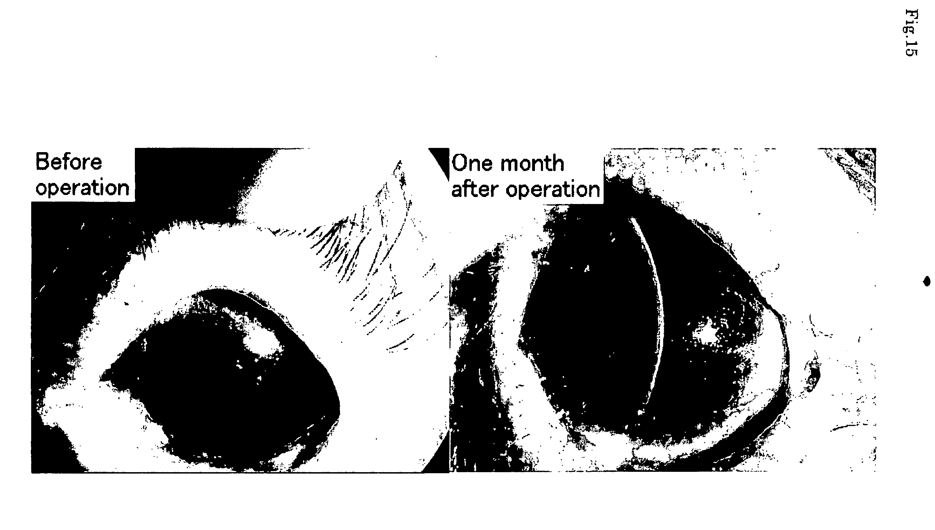

Fig. 15 shows the states of the ocular surface before the operation (left image) and one month after the operation (right image) of a patient with ocular cicatricial pemphigoid. It is known that when a general operation for cataract is carried out for a patient with ocular cicatricial pemphigoid, from the time of the operation, the conjunctiva invades to form cicatrical tissue, thereby causing significant haze in cornea. In this case, eyeball adhesion due to the ocular cicatricial pemphigoid was observed in the anterior part, however, one month after the operation, the state of the ocular surface was stable. The visual acuity is recovered from 0.06 before the operation to 0.6 at the present time.

From the above-mentioned results, the effectiveness of the corneal epithelium-like sheet (corneal epithelium transplantation sheet) to a human was demonstrated.

The present invention is not limited to the above-mentioned description of the embodiments and examples. Changes and variations may be made without departing from the spirit or scope of the following claims and in a range where person skilled in the art can easily achieve.

Hereinafter, the following matters are disclosed.
(11) A method of constructing a corneal epithelium transplantation sheet, the method including; a) culturing oral mucosal epithelial cells on a collagen layer; and b) when the oral mucosal epithelial cells are proliferated and a layered structure of the cells is formed, bringing the outermost layer into contact with the air.
(12) The method of constructing a corneal epithelium transplantation sheet described in (11), wherein the step a) is carried out in coexistence of supporter cells.
(13) The method of constructing a corneal epithelium transplantation sheet described in (11), wherein the step a) is carried out in coexistence of supporter cells and in a state in which an isolation membrane with pore size through which the supporter cells cannot pass exists between the supporter cell and the collagen layer.
(14) The method of constructing a corneal epithelium transplantation sheet described in any one of (11) to (13), wherein the collagen layer is derived from amniotic membrane.
(15) The method of constructing a corneal epithelium transplantation sheet described in any one of (11) to (13), wherein the collagen layer consists of amniotic membrane from which the epithelium has been removed.
(16) A method of constructing a corneal epithelium transplantation sheet, including the steps of:
   inoculating supporter cells in a first container to form a supporter cell layer;
   setting a second container, which has a bottom face made of an isolation membrane with pore size through which the supporter cells cannot pass, in the first container so that the bottom face is located in a culture medium;
   forming a collagen layer on the bottom face of the second container;
   inoculating oral mucosal epithelial cells on the collagen layer;
   culturing the oral mucosal epithelial cells to form a layered structure of the cells; and
   bringing the outermost layer of the layered structure of the oral mucosal epithelial cells into contact with the air.
(17) The method of constructing a corneal epithelium transplantation sheet described in (16), wherein the collagen layer is derived from the amniotic membrane.
(18) The method of constructing a corneal epithelium transplantation sheet described in (16), wherein the collagen layer consists of amniotic membrane from which the epithelium has been removed.

### INDUSTRIAL APPLICABILITY

Hitherto, the ocular surface reconstruction operation has included: collecting corneal epithelium stem cells from autologous (auto) or other person (allo); and transplanting them to a patient. However, collecting the stem cells from the autologous normal eye may cause exhaustion of stem cells in the normal eye in the future. Furthermore, in the transplantation of stem cells from the other person, the risk of immunological rejection may be usually accompanied. In addition, there is a problem as to a shortage of donor. According to the corneal epithelium-like sheet and the corneal epithelium transplantation sheet, since autologous oral mucosal epithelium can be used as a cell source for a transplantation material, there is no risk of causing the exhaustion of stem cells, and there is extremely small risk of immunological rejection. Furthermore, transplantation operation can be carried out without worrying about the shortage of donor. In particular, in patients from which autologous corneal epithelium stem cells are difficult or impossible to be collected, that is, in a patient with bilateral-eye corneal disease, the present invention is significant because a transplantation material constructed by using an autologous cell can be used for transplantation.

The corneal epithelium-like sheet (corneal epithelium transplantation sheet) of the present invention has excellent take after transplantation, and has adhesion sufficient enough to resist blinking. Furthermore, in the basal cell layer, predetermined proliferation property is maintained, while an epithelium-like cell layer in which cells are layered and differentiated in the vertical direction is formed and has and the epithelium-like cell layer has a structure that is extremely similar to that of the corneal epithelium. Furthermore, also a barrier function necessary for corneal epithelium to exert the function is provided. Furthermore, the transparence is high. Thus, the corneal epithelium-like sheet (corneal epithelium transplantation sheet) of the present invention is extremely excellent as a transplant material for reconstructing the corneal epithelium.

## Claims

1. A corneal epithelium-like sheet, comprising a cell layer in which a layered structure of cells derived from oral mucosal epithelial cells is formed.

2. The corneal epithelium-like sheet according to claim 1, wherein the cells of the outermost layer are not cornified, and/or are flat-shaped.

3. The corneal epithelium-like sheet according to claim 1 or 2, wherein the corneal epithelium-like sheet has a barrier function.

4. A corneal epithelium transplantation sheet comprising a cell layer as defined in claim 1 or 2, in which a layered structure of cells is formed on a collagen layer.

5. The corneal epithelium transplantation sheet according to claim 4, wherein said sheet has a barrier function.

6. A method of constructing a corneal epithelium-like sheet, the method comprising:
a) culturing oral mucosal epithelial cells on a collagen layer; and
b) when the oral mucosal epithelial cells are proliferated and a layered structure of the cells is formed, bringing the outermost layer into contact with the air.

7. The method according to claim 6, wherein step a) is carried out in coexistence of supporter cells.

8. The method according to claim 6 or 7, wherein step a) is carried out in coexistence of supporter cells and in a state in which an isolating membrane with pore size through which the supporter cells cannot pass exists between the supporter cells and the collagen layer.

9. The method of claim 6, comprising the steps of:
(i) inoculating supporter cells in a first container to form a supporter cell layer;
(ii) setting a second container, which has a bottom face made of an isolation membrane with pore size through which the supporter cells cannot pass, in the first container so that the bottom face is located in a culture medium;
(iii) forming a collagen layer on the bottom face of the second container;
(iv) inoculating oral mucosal epithelial cells on the collagen layer;
(v) culturing the oral mucosal epithelial cells to form a layered structure of the cells; and
(vi) bringing the outermost layer of the layered structure of the oral mucosal epithelial cells into contact with the air.

10. The corneal epithelium transplantation sheet of claim 4 or 5 or the method according to any one of claims 7 to 9, wherein the collagen layer is derived from amniotic membrane, and/or consists of amniotic membrane from which the epithelium has been removed.

## Patentansprüche

1. Korneaepithel-ähnliches Blatt, umfassend eine Zellschicht, in welche eine geschichtete Struktur aus von Mundschleimhautepithelzellen abgeleiteten Zellen ausgebildet ist.

2. Korneaepithel-ähnliches Blatt nach Anspruch 1, wobei die Zellen der äußersten Schicht nicht verhornt und/oder flach geformt sind.

3. Korneaepithel-ähnliches Blatt nach Anspruch 1 oder 2, wobei das Korneaepithelähnliche Blatt eine Barrierefunktion aufweist.

4. Korneaepithel-Transplantationsblatt umfassend eine Zellschicht wie in Anspruch 1 oder 2 definiert, in welche eine Schichtstruktur aus Zellen auf einer Kollagenschicht ausbildet ist.

5. Korneaepithel-Transplantationsblatt nach Anspruch 4, wobei das Blatt eine Barrierefunktion aufweist.

6. Verfahren zur Herstellung eines Korneaepithel-ähnlichen Blattes, das Verfahren umfasst:
a) Kultivieren von Mundschleimhautepithelzellen auf einer Kollagenschicht; und
b) wenn die Mundschleimhautepithelzellen proliferierren und diese Zellen eine Schichtstruktur ausbilden, das Inkontaktbringen der äußersten Schicht mit der Luft.

7. Verfahren nach Anspruch 6, wobei Schritt a) in der Koexistenz von Trägerzellen erfolg.

8. Verfahren nach Anspruch 6 oder 7, wobei Schritt a) in der Koexistenz von Trägerzellen und in einem Zustand erfolgt, in welchem eine isolierende Membran mit einer Porengröße, die die Trägerzellen nicht passieren können, zwischen den Trägerzellen und der Kollagenschicht vorhanden ist.

9. Verfahren nach Anspruch 6, umfassend die Schritte von:
(i) Animpfen der Trägerzellen in einem ersten Container um eine Trägerzellschicht auszubilden;
(ii) Einsetzen eines zweiten Containers, welcher eine Unterseite aus einer Isolationsmembran mit einer Porengröße, die die Trägerzellen nicht passieren können aufweist, in den ersten Container sodass die Unterseite sich im Zellkulturmedium befindet;
(iii) Ausbilden einer Kollagenschicht auf der Unterseite des zweiten Containers;
(iv) Animpfen der Mundschleimhautepithelzellen auf die Kollagenzellschicht:
(v) Kultivieren der Mundschleimhautepithelzellen um eine Schichtstruktur der Zellen auszubilden; und
(vi) Inkontaktbringen der äußersten Schicht von der Schichtstruktur der Mundschleimhautepithelzellen mit der Luft.

10. Korneaepithel-Transplantationsblatt nach Anspruch 4 oder 5 oder das Verfahren nach einem der Ansprüche 7 bis 9, wobei die Kollagenschicht von einer Amnionmembran abgeleitet ist und/oder aus einer Amnionmembran besteht, von welcher das Epithel entfernt worden ist.

## Revendications

1. Feuillet de type épithélium cornéen, comprenant une couche de cellules dans laquelle est formée une structure en feuillets de cellules dérivées des cellules épithéliales de la muqueuse orale.

2. Feuillet de type épithélium coréen selon la revendication 1, dans lequel les cellules de la couche la plus externe ne sont pas cornées, et/ou sont de forme plate.

3. Feuillet de type épithélium cornéen selon la revendication 1 ou 2, le feuillet de type épithélium cornéen ayant une fonction de barrière.

4. Feuillet de transplantation de l'épithélium cornéen comprenant une couche de cellules telle que définie dans la revendication 1 ou 2, dans lequel est formée une structure en feuillets de cellules sur une couche de collagène.

5. Feuillet de transplantation de l'épithélium cornéen selon la revendication 4, ledit feuillet ayant une fonction de barrière.

6. Pocédé de construction d'un feuillet de type épithélium cornéen, le procédé comprenant les étapes consistant à:
a) mettre en culture des cellules épithéliales de la muqueuse orale sur une couche de collagène; et
b) lorsque les cellules épithéliales de la muqueuse orale ont proliféré et qu'une structure en feuillets des cellules s'est formée, mettre en contact la couche la plus externe avec l'air.

7. Procédé selon la revendication 6, dans lequel l'étape a) est réalisée conjointement avec des cellules de support.

8. Procédé selon la revendication 6 ou 7, dans lequel l'étape a) est réalisée conjointement avec des cellules de support et de manière à ce qu'il existe entre les cellules de support et la couche de collagène une membrane isolante ayant une taille de pores empêchant les cellules de support de passer à travers celle-ci.

9. Procédé selon la revendication 6, comprenant les étapes consistant à:
(i) inoculer des cellules de support dans un premier conteneur afin de former une couche de cellules de support;
(ii) déterminer un second conteneur, qui a une face inférieure constituée d'une membrane d'isolement ayant une taille de pores empêchant les cellules de support de passer à travers celle-ci, dans le premier conteneur, de sorte que la face inférieure se situe dans un milieu de culture;
(iii) former une couche de collagène sur la face inférieure du second conteneur;
(iv) inoculer des cellules épithéliales de la muqueuse orale sur la couche de collagène;
(v) mettre en culture les cellules épithéliales de la muqueuse orale afin de former une structure en feuillets des cellules; et
(vi) mettre en contact la couche externe de la structure en feuillets des cellules épithéliales de la muqueuse orale avec l'air.

10. Feuillet de transplantation de l'épithélium cornéen selon la revendication 4 ou 5 ou procédé selon l'une quelconque des revendications 7 à 9, dans lesquels la couche de collagène est dérivée d'une membrane amniotique, et/ou consiste en une membrane amniotique de laquelle a été éliminé l'épithélium.
